(19) Europäisches Patentamt / European Patent Office / Office européen des brevets

(11) **EP 1 908 441 B1**

(12) **EUROPEAN PATENT SPECIFICATION**

(45) Date of publication and mention
of the grant of the patent:
**08.04.2015 Bulletin 2015/15**

(21) Application number: **06766647.9**

(22) Date of filing: **13.06.2006**

(51) Int Cl.:
*A61F 13/15* (2006.01)    *A61F 13/49* (2006.01)
*A61F 13/539* (2006.01)    *A61F 13/495* (2006.01)
*A61F 13/512* (2006.01)    *A61F 13/513* (2006.01)
*A61F 13/537* (2006.01)

(86) International application number:
**PCT/JP2006/311836**

(87) International publication number:
**WO 2006/134918 (21.12.2006 Gazette 2006/51)**

(54) **ABSORBENT ARTICLE**

SAUGFÄHIGER ARTIKEL

ARTICLE ABSORBANT

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **16.06.2005 JP 2005176861
16.06.2005 JP 2005176862
30.09.2005 JP 2005288177**

(43) Date of publication of application:
**09.04.2008 Bulletin 2008/15**

(73) Proprietor: **DAIO PAPER CORPORATION
Shikokuchuo-shi,
Ehime 799-0492 (JP)**

(72) Inventors:
• **EBITSUKA, Keisuke
c/o ELLEAIR PAPER TECH. CO., LTD
Sakura-shi,Tochigi 3291411 (JP)**

• **IDO, Yasuo
c/o ELLEAIR PAPER TECH. CO., LTD.
Sakura-shi, Tochigi 3291411 (JP)**
• **HORII, Hiroki
c/o ELLEAIR PAPER TECH. CO., LTD.
Sakura-shi, Tochigi 3291411 (JP)**
• **MURAKAMI, Kazuhisa
ELLEAIR PAPER TECH. CO., LTD.
Sakura-shi, Tochigi 3291411 (JP)**

(74) Representative: **Held, Stephan et al
Meissner, Bolte & Partner GbR
Postfach 86 06 24
81633 München (DE)**

(56) References cited:
**EP-A2- 1 138 301        JP-A- 62 125 001
JP-A- 2004 298 454        US-A- 5 643 240**

**Description**

Technical Field

The present invention relates to an absorbent article such as a disposable diaper or an absorbent pad. In particular, the invention relates to an absorbent article having improved performance of absorbing and retaining soft feces.

Background Art

In this kind of absorbent articles, there has been hitherto a problem in the performance of absorbing and retaining soft feces. Specifically, there occur situations where soft feces remain on a surface sheet without passing through the surface sheet, or where soft feces that have passed through the surface sheet is not held by an absorbent but flow back to the top of the surface sheet, thereby causing rashes on the skin or requiring troublesome works of skin wiping.

From this point of view, in order to improve the capability of carrying away soft feces rapidly from the skin, as well as accommodating and retaining soft feces at a location distant from skin (may also be simply referred to as performance of absorbing and retaining soft feces and the like), there have been proposed a technology of facilitating the passage of soft feces by using a perforated sheet as the surface sheet, and a technology of forming a space for accommodating soft feces between a surface sheet and an absorber (see, for example, Patent Document 1, Patent Document 2, Patent Document 3 and Patent Document 4).

However, although the system of Patent Document 2 in particular, among others, is conceived to have excellent performance of absorbing and retaining soft feces, according to the experiment conducted by the inventors of the present invention, it is difficult with hydrophilic continuous fibers to transfer soft feces sufficiently to the absorber, and further improvement is desired for the performance of absorbing and retaining soft feces.

Further improvement is also desired in the performance of permeating soft feces (capability of carrying away soft feces rapidly from the skin), since there occur situations such as that soft feces locally remain on the surface sheet, and the like.

Moreover, since soft feces are viscous unlike urine or the like, there has been a risk that the soft feces which have passed through the surface sheet (particularly, the liquid portion), form a film (barrier) on the surface of the crepe paper or the like of a forehead wrapping which wraps an absorber, thereby causing a flowback of urine, soft feces or the like. Accordingly, there is a demand for the development of a technology capable of transferring soft feces surely as well as rapidly to the absorber.

[Patent Document 1] Japanese Patent Application Laid-Open No. 2-65861
[Patent Document 2] Japanese Patent Application Laid-Open No. 2001-276125
[Patent Document 3] Japanese Patent Application Laid-Open No. 2001-269362
[Patent Document 4] United States Patent Patent No. 5,643,240

Disclosure of the Invention

Problems to be Solved by the Invention

A primary object to be addressed by the present invention is to provide an absorbent article having an excellent performance of absorbing and retaining urine or soft feces.

Means for Solving the Problems

The present invention, which addresses this object, is as follows.

[Invention of Claim 1]

An absorbent article including in sequence, a perforated surface sheet facing the skin, when in use, and allows permeation of excremental liquid therethrough; a permeation sheet which allows permeation of excremental liquid therethrough; and an absorbent element disposed on a backside of the permeation sheet which includes cotton-like pulp for retaining the excremental liquid,
wherein a soft feces permeation speed S1 of the surface sheet is higher than a soft feces permeation speed S2 of the permeation sheet;
the perforated surface sheet has a group of permeation holes for excremental liquid formed at an excretion region thereof where at least an area that faces, when in use, the body part excreting the excremental liquid and its vicinity are included, and an effective opening area of a single permeation hole is 3 to 75 mm$^2$, and a ratio of opening area is 10 to 80%; and the absorbent element has a cotton-like pulp layer exposed on a use side thereof, and the surface sheet, the permeation sheet and the cotton-like pulp layer of the absorbent element are directly adjacent in sequence.

The article has a main body section which includes the permeation sheet, the absorbent element and a fusion section formed by fusing the surface sheet onto a top surface of the main body section, wherein the fusion section is provided at at least one site in the width direction at various locations in the front and rear directions of the article.

The fusion section is formed to have a longer length in the MD direction and in the CD direction, compared to the permeation holes of the surface sheet.

[Invention of Claim 2]

The absorbent article according to claim 1, wherein the soft feces permeation speed S2 of the permeation sheet is greater than a soft feces permeation speed S3 of the cotton-like pulp layer of the absorbent element.

(Operating Effects of Claims 1 and 2)

According to the present invention, the soft feces permeation speed S1 of the surface sheet is greater than the soft feces permeation speed S2 for the permeation sheet. In a more preferred embodiment, the soft feces permeation speed S2 is to be greater than the soft feces permeation speed S3 of the cotton-like pulp layer of the absorbent element. Thus, the permeations speeds are after all correlated in the relationship of S1 > S2 > S3.

As described above, absorbent articles of this kind are required to have a good capability of carrying away soft feces rapidly from the skin as well as accommodating and retaining the soft feces at a location distant from the skin. It has been found that in order to achieve this, each of the constituent elements not only needs to have a high soft feces permeation speed, but also needs to have a speed gradient as defined in the present invention with regard to the soft feces permeation speed for each of the constituent elements, and the speed gradient is dominant to the absolute value of the speed.

By means of the soft feces permeation speed gradient of the present invention, the performance of absorbing and retaining urine or soft feces is made excellent.

Furthermore, it has been found that it is important to transfer the soft feces rapidly to the permeation sheet through the surface sheet, and for this reason, a perforated sheet is used as the surface sheet, while particularly for the permeation holes for excremental liquid formed at least in an excretion region which includes the area of the perforated surface sheet facing the body part excreting the excremental liquid, and the vicinities, it is required that the effective opening area of a single permeation hole be 3 to 75 mm$^2$, and the ratio of opening area be 10 to 80%. Once the soft feces are allowed to permeate rapidly through the surface sheet to be transferred to the permeation sheet, then it becomes difficult for the flowback to the surface side to occur thereafter, under the situation where the relationship of S1 > S2 is effective.

In this case, if the excremental liquid cannot be transferred rapidly to the absorbent element, the excremental liquid eventually stays behind in the permeation sheet. In the case of using an absorber which is made up mainly of cotton-like pulp, it is conventional to wrap around the absorber with tissue paper so as to maintain the shape. However, since the liquid portion of soft feces is more viscous, unlike urine, the permeability is poorer. If the surface side of the absorber is more difficult for the liquid portion of the soft feces to permeate through, the surface side becomes a barrier to the liquid portion of the soft feces that are to be transferred from the permeation sheet, thereby making it difficult for the soft feces to permeate through the permeation sheet.

In this way, the performance of absorbing and retaining soft feces becomes insufficient. Thus, by exposing the cotton-like pulp layer directly to the side of use-surface beneath the permeation sheet, without inserting another sheet between the permeation sheet and the absorbent element, the excremental liquid transferred to the permeation sheet can be rapidly transferred to the cotton-like pulp layer, and accordingly, the tendency of the excremental liquid staying behind in the permeation sheet can be prevented. As a result, the performance of absorbing and retaining soft feces is enhanced.

Conventional absorbent articles in general include a liquid-permeable surface sheet facing the skin, and a main body section for receiving the excremental liquid which has permeated through the surface sheet, and the surface sheet is fused to the surface of the main body section by heat-sealing, ultrasonic sealing or the like. This fusion section has been hitherto provided over the entire surface sheet, but there have existed sections having no fusion section anywhere in the width direction with respect to the anteroposterior direction of the article. As a result, when an absorbent article is bent along the anteroposterior direction of the body, crimps are generated on the surface sheet depending on the present or absence of the fusion section, and the permeation performance of the surface sheet is deteriorated.

The present invention has been achieved based on such finding, and by providing a fusion section at at least one site in the width direction at various locations in the anteroposterior direction of the article, the fusion strength and hardening due to fusion become uniform along the anteroposterior direction of the article, and the surface and the main body section feel more like being unified. Thus, crimps are hardly generated even though the article is bent along the anteroposterior direction of the body. As a result, the permeation performance inherent to the surface sheet can be exhibited to the maximum, and thus the permeation performance can be further improved as compared with the products of prior art.

Although the position of the fusion section and the position of permeation holes partially overlap, the fusion section can be definitely formed by processing. As a result, generation of crimps can be certainly prevented, and the permeation performance can be certainly improved. Additionally, the MD direction and the CD direction mean the directions in the fusion processing.

[Invention of Claim 3]

The absorbent article according to claim 1 or 2, wherein the permeation sheet is made mainly of water-repellent or hydrophobic fibers.

(Operating Effect of Claim 3)

If the permeation sheet is hydrophilic, the permeability would be excellent in the case of urine. However, in the case of excremental liquid arriving at the permeation sheet, for example, in the case of soft feces, the solid portions of the soft feces are retained between the fibers, and in association therewith, the liquid portion in the soft feces or between the solid portions is also retained. Thus, eventually the liquid portion of the soft feces is brought to the state of being retained and remained behind, thereby it becoming difficult for the liquid portion to be transferred to the absorbent element.

In this regard, when the permeation sheet uses a sheet formed mainly from water-repellent or hydrophobic fibers, it is conceived that even if much of the solids of the soft feces is retained between the fibers, the water-repellent or hydrophobic fibers will exhibit a function of manifesting filterability against the liquid portion in the soft feces or between the solid portions, and therefore, the liquid portion is separated from the solid portions and transferred to the absorbent element side. As a result, since the aforementioned permeation sheet has a room to continually receive soft feces, and its function of continuous filtration is manifested, the liquid portion of the soft feces can be transferred to the absorbent element side rapidly in bulk.

[Invention of Claim 4]

The absorbent article according to any one of claims 1 to 3, wherein the permeation sheet comprises a filament assembly, and filaments of the filament assembly are oriented in the planar direction of the sheet.

(Operating Effect of Claim 4)

If the permeation sheet is formed from filament assemblies, and the filaments are oriented in the planar direction of the sheet, the permeability of soft feces, particularly of the liquid portion, becomes excellent. The reason for this is conceived to be based on the following phenomenon. Specifically, in the case of random non-woven fabrics of short fibers, there is limitation even in increasing or decreasing the fiber density, in order to maintain the shape of the sheet. Thus, it is difficult to transfer the solid portions of soft feces to the permeation sheet. In this respect, if filament assemblies are used instead of short fiber assemblies, the solid portions of soft feces can be transferred between filaments. Furthermore, if the direction of orientation of the filaments is in the thickness direction as in the case of Patent Document 2, unless the solid portions and the liquid portion of the soft feces slip through between the filaments, it is difficult to transfer the liquid portion to the absorbent element side. However, if the filaments are oriented in the planar direction of the sheet as in the case of the present invention, the solid portions of the soft feces are retained and remained behind between the filaments, and the filaments function as a filter material. Thus, the liquid portion of the soft feces can be transferred to the absorbent element side rapidly in bulk.

In addition, as for the filaments, spirally crimped filaments can be used. If the filaments are spirally crimped, the filaments exhibit the function as a filter material more effectively, and the liquid portion of soft feces can be transferred to the absorbent element side rapidly in bulk.

[Invention of Claim 5]

The absorbent article according to any one of claims 1 to 4, wherein an area ratio of the fusion section is lower in the middle part between front and back edges than that at the front and back edges of the article.

(Operative Effect of Claim 5)

In the present invention, the fusion section is disposed densely along the anteroposterior direction of the article. However, since the fusion section practically does not allow the permeation of excremental liquid, if the area is excessively increased, the permeation performance may be impaired. Therefore, it is preferable to construct as described in the present claim, so that the area ratio of the fusion section is lower in the middle part onto which excremental liquid is excreted.

[Invention of Claim 6]

The absorbent article according to claim 5, wherein the main body section is exposed at both lateral sides of the surface sheet, and a quasi-fusion section is formed on an exposed part of the main body section by subjecting an upper surface of the main body section to fusion-processing without involving the surface sheet.

(Operating Effect of Claim 6)

For example, when it becomes difficult for a low-viscosity liquid such as urine to permeate the surface sheet, such as in the case of making a surface sheet water-repellent so as to suppress diffusion on the surface sheet, or the like, it is preferable if the main body section is exposed at the lateral sides of the surface sheet, because excremental liquid can be directly introduced to the main body section from the lateral sides, without involving the surface sheet.

However, in this case, because the width of the surface sheet intended for fusion becomes narrow, if the area ratio of the fusion section in the middle part of the article is maintained low as described above, there may be a risk that abrupt changes occur in the fusion processing conditions (pressure, processing time, etc.) in the anteroposterior direction of the article, thus making stable and high-quality processing difficult.

Thus, in the invention according to the subject claim, although the main body section is exposed at lateral sides of the surface sheet, a quasi-fusion section is provided at the exposed parts, without decreasing the width intended for fusion in association with the exposure. Then, even if the area ratio of the fusion section of the surface sheet in the middle part is decreased in order to improve absorbability, the fluctuation in the fusion processing conditions may be suppressed to be low. Therefore, stable and high-quality fusion processing of the surface sheet can be performed, while facilitating the absorption of a low-viscosity liquid such as urine.

[Invention of Claim 7]

The absorbent article according to any one of claims 1 to 6, wherein the absorbent element has an absorber containing super absorbent polymer particles and an element for wrapping the absorber, and
at least part of an area of the element for wrapping the absorber located between the absorber and the permeation sheet is made of a non-woven fabric having a density of 0.01 to 0.2 g/cm$^3$.

[Invention of Claim 8]

The absorbent article according to any one of claims 1 to 7, wherein the absorbent element has an absorber containing super absorbent polymer particles and an element for wrapping the absorber, and
at least part of an area of the element for wrapping the absorber located between the absorber and the permeation sheet is made of an air-through non-woven fabric.

[Invention of Claim 9]

The absorbent article according to claim 7 or 8, wherein an area of the element for wrapping the absorber other than the area made of a non-woven fabric is made of tissue paper or crepe paper.

[Invention of Claim 10]

The absorbent article according to any one of claims 7 to 9, wherein the non-woven fabric has a fineness of 2 to 7 dtex and a basis weight of 10 to 45 g/m$^2$.

[Invention of Claim 11]

The absorbent article according to any one of claims 7 to 10, wherein the non-woven fabric comprises water-repellent or hydrophobic fibers.

[Invention of Claim 12]

The absorbent article according to any one of claims 7 to 11, wherein the absorber comprises an upper layer absorber and another layer absorber, and the upper layer absorber does not contain super absorbent polymer particles.

(Operating Effect of Claims 7 to 12)

The operating effect of claims 7 to 12 will be described later.

Effect of the Invention

According to the present invention, an absorbent article having excellent performance of absorbing and retaining urine or soft feces is obtained.

Best Mode for Carrying Out the Invention

[Embodiments of the Invention According to Claims 1 to 4]

Hereinafter, the embodiments of the invention described in claims 1 to 4 will be fixed up to the aspect of the use of underpants-type diaper for adults, and detailed explanation will be given with reference to an exemplary disposable absorbent pad. However, it is needless to say that the absorbent article of the present invention is applicable to various absorbent articles including the underpants-type diaper itself, a tape-type diaper and the like.

<Fundamental Form>

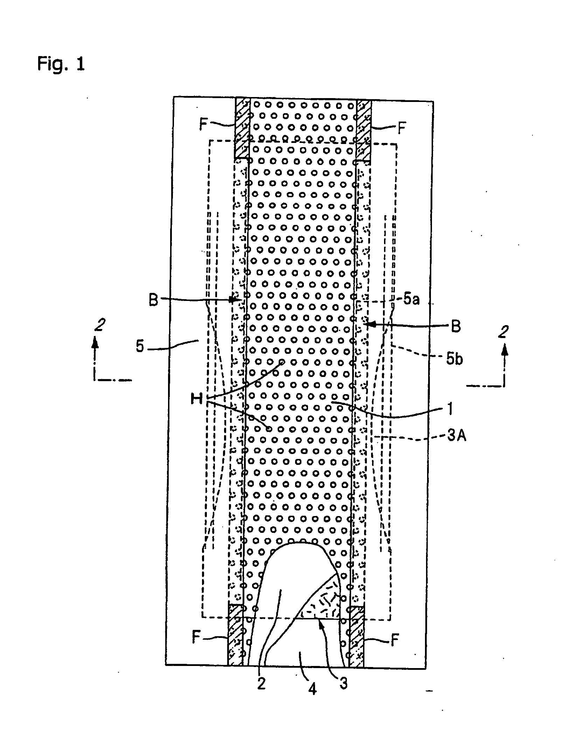

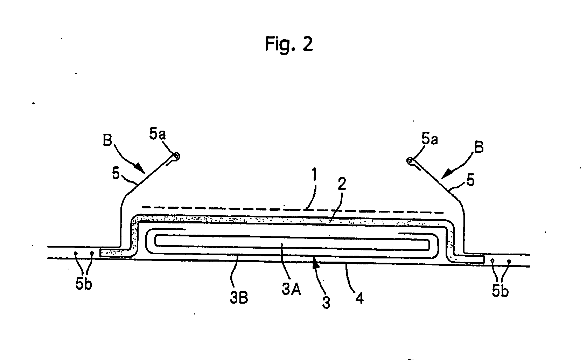

The absorbent pad shown in Fig. 1 and Fig. 2 includes, in sequence from the top side toward the backside, a surface sheet 1 facing the skin, which sheet allows excremental liquid to permeate through and comprises a perforated sheet; a permeation sheet 2 which allows excremental liquid to permeate through; and an absorbent element 3 which is disposed on the backside of the permeation sheet 2 and contains a cotton-like pulp layer retaining the excremental liquid. Furthermore, the absorbent pad also has barrier cuffs B, B standing from both sides of the product to the user-surface.

The excretion region which includes the area of the perforated surface sheet facing the body part excreting the excremental liquid, and the vicinities, has a large number of permeation holes H, H... for excremental liquid formed over the entire area in the present embodiment.

For the group of permeation holes H, H..., those having an effective opening area of a single permeation hole of 3 to 75 mm$^2$ (more preferably 5 to 30 mm$^2$), and a ratio of opening area of 10 to 80% (preferably 30 to 60%) are used. If the opening area and the ratio of opening area are small, the permeability of the solid portions of soft feces is deteriorated, whereas if the opening area is excessively large, the solid portions of soft feces remaining behind on the surface of the permeation sheet is contacted with the skin, thereby causing rashes on the skin or requiring troublesome works of skin wiping. If the ratio of opening area is excessively large, the strength of the system is decreased, which is undesirable.

The permeation sheet 2 which directly faces the surface sheet 1, functions as a second sheet in the depicted example. Details thereof will be described later.

The absorbent element 3 which directly faces the permeation sheet 2 and retains excremental liquid, has a cotton-like pulp layer 3A exposed to the side of use-surface. The specific example of Fig. 2 shows the constitution of an absorbent element 3 in which the backside and the lateral sides of the cotton-like pulp layer 3A are wrapped with the tissue paper or crepe paper (wrapping material 3B) of a forehead wrapping.

The backside sheet 4 which constitutes the back surface side of the absorbent article is a liquid-impermeable sheet in the embodiment, and is formed from, for example, a plastic sheet. The backside sheet 4 may be permeable to air. The backside sheet 4 is extended out from the side edges of the absorbent element 3 to the lateral direction.

At the lateral sides of the article, a barrier sheet 5 is provided. The outer side of the barrier sheet is fixed to the extended portion of the backside sheet by a hot melt adhesive or the like, and the tip side of the inner free part is folded toward the inside, with an elastic stretch member 5a such as rubber thread is fixed onto the folded part, while being stretched. Thus, upon wearing the article, the free part constitutes the standing barrier cuffs B, B. According to the present invention, it is not limited as to whether the barrier cuffs are present or not, but it is desirable to provide barrier cuffs in order to prevent side leakage of soft feces.

In the embodiment, the permeation sheet 2 is extended outwards from the side edges of the absorbent element 3, and the extended section is interposed between the barrier sheet 5 and the backside sheet 4, and fixed by a hot melt adhesive or the like.

The surface sheet 1 is not in a state of being fixed to the barrier sheet 5 and the backside sheet 4. The surface sheet 1 is needed to be fixed to the members of the article in any form. In the embodiment, the surface sheet 1 is fixed onto the permeation sheet 2. Upon this fixation, typically there is employed a configuration in which the surface sheet 1 is fixed onto the permeation sheet 2 by spiral coating with a hot melt adhesive or the like. However, the surface sheet 1 according to the present invention is a perforated sheet and has large openings. As a result, the hot melt adhesive protrudes from the permeation holes to the use-surface, and permeation of excremental liquid through the permeation holes H is impeded by the presence of the hot melt adhesive. Thus, to the extent that slackening of the surface sheet 1 does not occur (if the fixation sites are widely distributed, slackening of the surface sheet 1 occurs, thus causing separation of the two parts, and the transfer of excremental liquid to the permeation sheet 2 does not occur smoothly), the overall fixed area is kept as small as possible. Further, in order to prevent protrusion of the hot melt adhesive from the permeation holes to the use surface side, it is desirable to partially fix the surface sheet 1 onto the permeation sheet 2 by heat sealing or ultrasonic sealing.

Meanwhile, in order to make the barrier cuffs B rise at the middle part, but not at the front and back edge parts, the inner front and back edges of the barrier sheet 5 are fixed to the surface sheet 1 at four points in total, by a hot melt adhesive (the points of fixation are shown by symbol F).

Furthermore, elastic stretch members 5b, 5b... are fixed, while being stretched, between the barrier sheet 5 and the backside sheet 4, along the leg periphery, so that fittability of the article around the leg periphery is enhanced.

<Other embodiments>

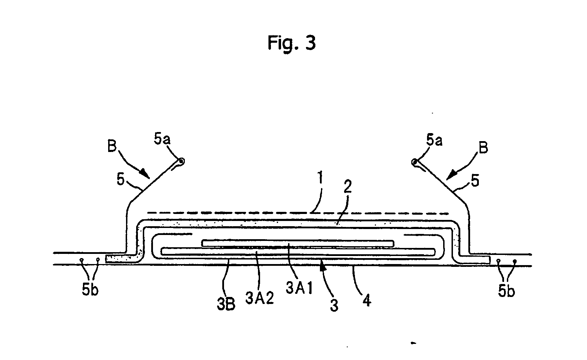

Subsequently, various other embodiments will be described. In Fig. 3, the cotton pulp layer is changed to a single layer of cotton-like pulp layer 3A, including an upper cotton-like pulp layer 3A1 having a narrow width and a lower cotton-like pulp layer 3A2 having a broad width.

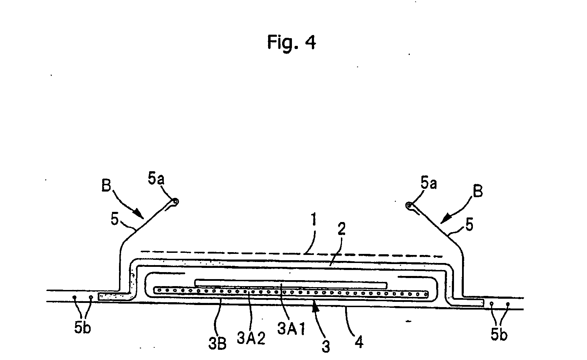

At an appropriate site beneath the permeation sheet 2, super absorbent polymer particles (SAP) can be provided in an appropriate area of the absorbent element 3 for the purpose of retaining large quantities of liquid portion. For example, the polymer particles can be dispersed within the absorber 3A, in addition to dispersing and spreading over the absorber 3A. Also, in the case of having a bilayer structure of the upper cotton-like pulp layer 3A1 and the lower cotton-like pulp layer 3A2, as shown in Fig. 4, super absorbent polymer particles can be incorporated only to the lower cotton-like pulp layer 3A2. The objects indicated with symbol O in fig. 4 are the super absorbent polymer particles.

According to the present invention, as long as the article includes the surface sheet, a permeation sheet, and absorbent element containing cotton-like pulp in this order, the configuration of wrapping the lateral sides of the sheets may be exemplified by a configuration in which the backside sheet wraps up to the two lateral sides above the surface sheet by forehead wrapping; a configuration in which the permeation sheet wraps up the entire absorbent element; a configuration in which the surface sheet wraps up the permeation sheet and an absorbent element; a configuration in which the permeation sheet wraps up the entire absorbent element, and the surface sheet further wraps up the permeation sheet and the entire absorbent element from the above; or the like may be employed.

<Soft feces permeation speed>

According to the invention, it is preferable that the soft feces permeation speed S1 of the surface sheet 1, the soft feces permeation speed S2 of the permeation sheet 2, and the soft feces permeation speed S3 of the top layer of the absorbent element (in the case of having a cotton-like pulp layer 3A, that cotton-like pulp layer 3A) are in the relationship of

$$S1 > S2 > S3.$$

As for the method of measuring the permeation speed of soft feces, the following Example of using pseudo-soft feces may be referred to.

<Configuration for fixation of surface sheet to permeation sheet>

Meanwhile, during the production, it is preferable to fix the surface sheet 1 to the permeation sheet 2 by fusion, as previously explained in the Summary. As for this method of fusion, heat sealing or ultrasonic sealing can be used. These process methods are the same in the fundamental principle, except the difference in the manner of heating, and fusion is performed such that a number of projections provided on the outer periphery of a processing roll are heated while being pressed on the surface sheet 1, thus to fuse the pressed part generated by the projections.

In a preferred configuration, these fusion sections m, m... are provided substantially at at least one site in the width direction at various locations in the anteroposterior direction of the product. Such pattern is arranged such that the projections of the processing roll are substantially not discontinued in the direction of outer circumference. In other words, the pattern can be achieved by arranging the projections such that some number of projections always performs fusion.

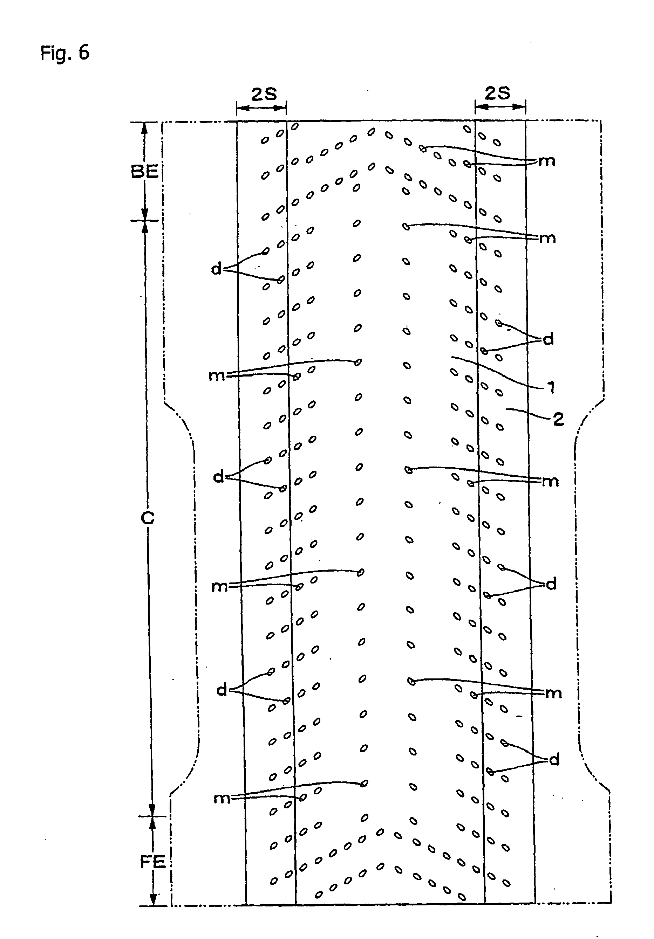

The pattern of the fusion sections m, m... can be appropriately determined, but for example, a pattern in which multiple groups of fusion sections comprising a plurality of fusion sections m, m... arranged in a V-shaped line, at different locations in the anteroposterior direction as well as in the width direction, are provided in parallel along the anteroposterior direction as shown in Fig. 6, can be used. As for the configuration of the fusion sections m, m..., an appropriate configuration such as an elliptical shape, a round shape, a triangular shape, a rectangular shape, a diamond shape or a hexagonal shape, can be employed, and these can be arranged regularly or irregularly. Furthermore, the fusion section m in the drawing indicates the shape of the processing range; therefore, since the surface sheet 1 is perforated, there are cases in which the shape of the fusion section does not match the shape of the bonding part where fusion actually occurs.

With regard to the front edge part FE and the back edge part BE of the article, and to the middle part C between the edges, the area ratios of the fusion sections m, m... (ratio of the area occupied by the entire fusion sections per unit area) may be made equal, or the area ratio of the latter may be made higher. However, since permeation of excremental liquid is difficult in the fusion sections m, m..., it is preferable to lower the area ratio of the fusion sections m, m... at the middle part C between the edge parts, compared to the area ratios at the front and back edge parts FE and BE of the article, in order not to impair the permeability of the surface sheet 1. The area ratio of the fusion sections m, m... can be adjusted by reducing the number of the fusion sections m, m... as shown in the depicted example, as well as can be adjusted by reducing the area per one site of the fusion section m. In addition, the lengths of the front and back edge parts FE and BE, and the middle part C of the article can be appropriately set, but for example, the front and back edge parts FE and BE can be respectively set to be in the range of 1 to 100 mm from the front tip and the back tip, and the area between the two can be taken as the middle part C.

The area per one site of the fusion section m is preferably in the range of 1 to 300 mm$^2$, and particularly in the range of 5 to 30 mm$^2$, and the area ratio is preferably in the range of 0.1 to 20%, and particularly in the range of 0.5 to 10%. In the case where the area ratio of the fusion sections m, m... for the middle part C in the anteroposterior direction is set to be lower than those for the front and back edge parts FE and BE, the area ratio for the front and back edge parts FE and BE is preferably in the range of 5 to 30%, and particularly in the range of 5 to 20%, while the area ratio for the middle part C is preferably in the range of 1 to 30%, and particularly in the range of 1 to 15%.

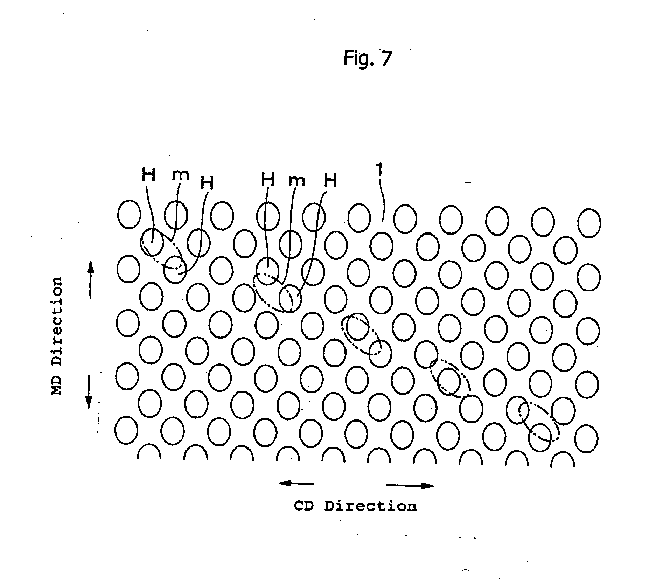

Furthermore, in the case where the surface sheet 1 is a perforated sheet, it is preferable to select the size such that the lengths of the fusion sections m, m... in the MD direction (the longitudinal direction of the product) and in the CD direction (the transverse direction of the product) are longer compared to the permeation holes H of the surface sheet 1. Then, as shown in Fig. 7, even though the positions of the fusion sections m, m... and the positions of the permeation holes H partly overlap, the fusion sections m, m... can be definitely formed by processing, which is preferable.

[Embodiments of the Invention According to Claims 5 to 8]

Next, the embodiments of the invention according to claims 5 to 8 will be described in detail with reference to an example of a disposable absorbent pad which is fixed up on the use-surface of an underpants-type diaper for adults. However, it is needless to say that the present invention is also applicable to various absorbent articles such as underpants-type diapers and tape-type diapers, regardless of whether the article is for adults or infants.

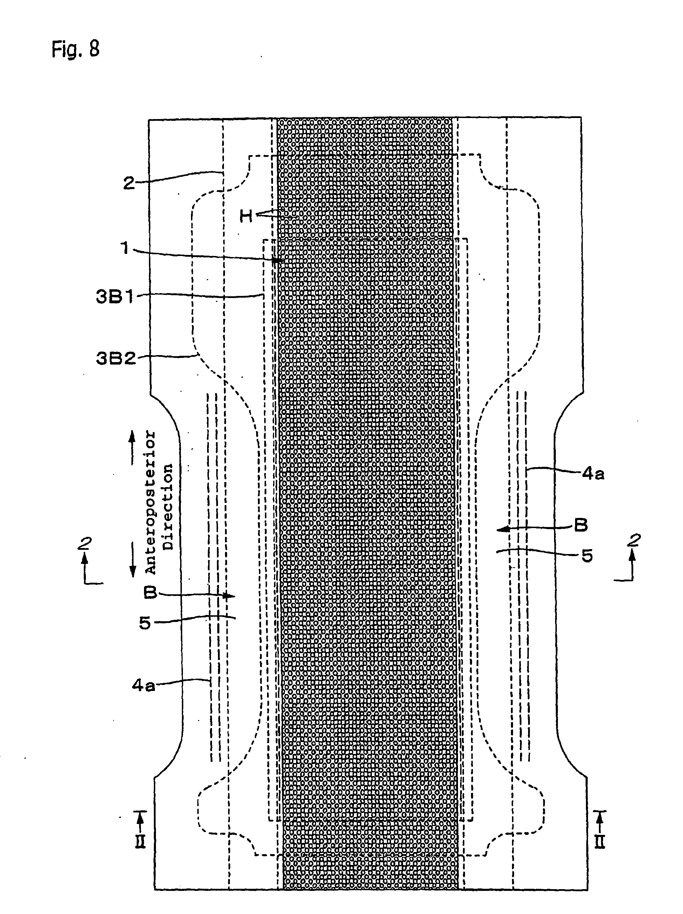

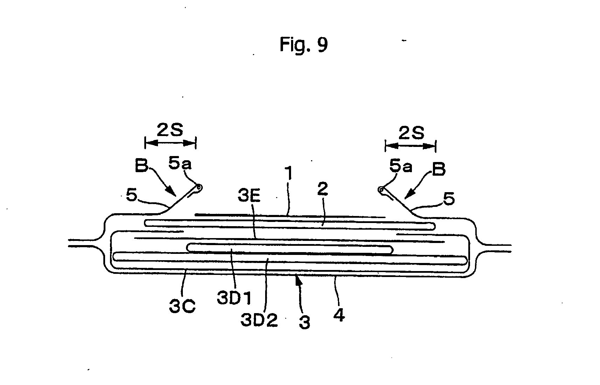

Fig. 8 and Fig. 9 show examples of the absorbent pad according to the present invention, and the absorbent pad includes a main body section comprising, in sequence from the top side toward the backside, a surface sheet 1 facing the skin, which sheet allows excremental liquid to permeate through; a permeation sheet 2 which allows excremental liquid to permeate through; and an absorbent element 3 which retains the standing from the top surface side.

The surface sheet 1 has a large number of permeation holes H, H... for excremental liquid formed at least in the area facing the anus, or over the entire area according to the embodiment. A surface sheet 1 which can be favorably used is one having an effective opening area of a single permeation hole of 3 to 75 mm$^2$, and having a ratio of opening area of 10 to 80%, with regard to a group of permeation holes for excremental liquid formed at least in the area facing the anus.- If the opening area and the ratio of opening area are small, the permeability of the solid portions of soft feces is deteriorated, while if the opening area is excessively large, the solid portions of soft feces remaining behind on the surface of the permeation sheet 2 is contacted with the skin, thereby causing rashes on the skin or requiring troublesome works of skin wiping. If the ratio of opening area is excessively large, the strength of the system is decreased, which is undesirable.

For example, if the surface sheet 1 is made water-repellent so as to suppress diffusion on the surface sheet 1, or the like, it becomes difficult for a low viscosity liquid such as urine to permeate through the surface sheet 1. In this case; as shown in the depicted example, it is preferable to allow the main body section to be exposed (protruded) from both lateral sides of the surface sheet 1 by making the width of the surface sheet 1 narrower than the width between the barrier cuffs B, B (if no barrier cuffs are available, the width of the main body section, and in this case, the width of the permeation sheet 2). Then, the excremental liquid can be directly guided from the two lateral sides to the main body section, without passing through the surface sheet 1.

The absorbent element 3 directly facing the permeation sheet 2 has absorbers 3D1 and 3D2, which are mainly composed of cotton-like pulp retaining excremental liquid; an upper wrapping material 3E covering the lateral sides of the permeation sheets 2 of the absorbers 3D1 and 3D2; and a lower wrapping material 3C which is composed of tissue paper or crepe paper covering the remaining surfaces of the absorbers 3b1 and 3B2. Thus, the absorbent element has a structure in which the absorbers 3B1 and 3B2 are wrapped by the upper wrapping material 3E and the lower wrapping material 3C.

As for the absorber, as shown in Fig. 8 and Fig. 9, it is preferable to use a bilayer absorber formed by laminating an upper absorber 3D1 with a narrow width over a lower absorber 3D2 with a broad width, but a single layer absorber 3D as shown in Fig. 10 can also be used.

The backside sheet 4 is extended from the side edges of the absorbent element 3 to the lateral sides, and reaches the top surface side while being folded, thus to form flaps which cover the lateral sides of the surface sheet 1.

At the lateral sides of the backside sheet 4 is provided a barrier sheet 5. The outer side of the barrier sheet is fixed to the extended portion of the backside sheet 4 by a hot melt adhesive or the like, and the tip side of the inner_free part is folded toward the inside, with an elastic stretch member 5a such as rubber thread is fixed onto the folded part, while being stretched. Thus, upon wearing the article, the free part constitutes the standing barrier cuffs B, B. According to the present invention, it is not limited as to whether the barrier cuffs are present or not, but it is desirable to provide barrier cuffs in order to prevent side leakage of soft feces.

Furthermore, an elastic stretch member 4a is fixed, while being stretched, between the barrier sheet 5 and the backside sheet 4, along the leg periphery, so that fittability of the article around the leg periphery is enhanced.

Meanwhile, during the production, the surface sheet 1 is fused to the main body section, that is, the permeation sheet 2 in the case of having the permeation sheet 2 as depicted in the diagram, and to the absorbent element 3 in the case of not having the permeation sheet 2. As for this method of fusion, heat sealing or ultrasonic sealing can be used. These process methods are the same in the fundamental principle, except the difference in the manner of heating, and fusion is performed such that a number of projections provided on the outer periphery of a processing roll are heated while being pressed on the surface sheet 1, thus to fuse the pressed part generated by the projections.

In the present invention, as shown in Fig. 6, these fusion sections m, m... are provided substantially at at least one site in the width direction at various locations in the anteroposterior direction of the product. Such pattern is arranged such that the projections of the processing roll are substantially not discontinued in the direction of outer circumference. In other words, the pattern can be achieved by arranging the projections such that some number of projections always performs fusion.

The pattern of the fusion sections m, m... can be appropriately determined, but for example, a pattern in which multiple groups of fusion sections comprising a plurality of fusion sections m, m... arranged in a V-shaped line, at different locations in the anteroposterior direction as well as in the width direction, are provided in parallel along the anteroposterior direction as depicted in the drawing, can be used. As for the configuration of the fusion sections m, m..., an appropriate configuration such as an elliptical shape, a round shape, a triangular shape, a rectangular shape, a diamond shape or a hexagonal shape, can be employed, and these can be arranged regularly or irregularly. Furthermore, the fusion section m in the drawing indicates the shape of the processing range; therefore, in the case where the surface sheet 1 or the main body section as the body to be fused (permeation sheet 2 in the present embodiment) is perforated, there are cases in which the shape of the fusion section does not match the shape of the bonding part where fusion actually occurs.

With regard to the front edge part FE and the back edge part BE of the article, and to the middle part C between the edges, the area ratios of the fusion sections m, m... (ratio of the area occupied by the entire fusion sections per unit area) may be made equal, or the area ratio of the latter may be made higher. However, since permeation of excremental liquid is difficult in the fusion sections m, m..., it is preferable to lower the area ratio of the fusion sections m, m... at the middle part C between the edge parts, compared to the area ratios at the front and back edge parts FE and BE of the article, in order not to impair the permeability of the surface sheet 1. The area ratio of the fusion sections m, m... can be adjusted by reducing the number of the fusion sections m, m... as shown in the depicted example, as well as can be adjusted by reducing the area per one site of the fusion section m. In addition, the lengths of the front and back edge parts FE and BE, and the middle part C of the article can be appropriately set, but for example, the front and back edge parts FE and BE can be respectively set to be in the range of 1 to 100 mm from the front tip and the back tip, and the area between the two can be taken as the middle part C.

The area per one site of the fusion section m is preferably in the range of 1 to 300 mm$^2$, and particularly in the range of 5 to 30 mm$^2$, and the area ratio is preferably in the range of 0.1 to 20%, and particularly in the range of 0.5 to 10%. In the case where the area ratio of the fusion sections m, m... for the middle part C in the anteroposterior direction is set to be lower than those for the front and back edge parts FE and BE, the area ratio for the front and back edge parts FE and BE is preferably in the range of 5 to 30%, and particularly in the range of 5 to 20%, while the area ratio for the middle part C is preferably in the range of 1 to 30%, and particularly in the range of 1 to 15%.

Furthermore, in the case where the surface sheet 1 is a perforated sheet, it is preferable to select the size such that the lengths of the fusion sections m, m... in the MD direction and in the CD direction are longer compared to the permeation holes H of the surface sheet 1. Then, as shown in Fig. 7, even though the positions of the fusion sections m, m... and the positions of the permeation holes H partly overlap, the fusion sections m, m... can be definitely formed by processing, which is preferable.

Furthermore, in the case where the area ratio of the fusion section m in the middle part C of the article is set to be lower than the area ratios at the front and back edge parts FE and BE, and the main body section (permeation sheet 2 in the present embodiment) is exposed at the two lateral sides of the surface sheet 1, as depicted in the drawing, it is preferable to perform the fusion processing over the entire exposed surface, including the exposed parts 2S of the main body section at the two lateral sides of the surface sheet 1, thereby to provide fusion sections m, m... on the surface sheet 1, and also to provide quasi-fusion sections d, d... on the surface of the main body section (permeation sheet 2 in the present embodiment) without involving the surface sheet 1. Then, as previously described, the surface sheet 1 can be fused stably with high quality.

[Embodiments of the Invention According to Claims 9 to 14]

Furthermore, the embodiments of the invention according to claims 9 to 14 will be explained in detail with reference to an example of a disposable absorbent pad which is fixed up on the use-surface of an underpants type diaper for adults. However, it is needless to say that the present invention is also applicable to various absorbent articles such as underpants-type diapers and tape-type diapers, regardless of whether the article is for adults or infants.

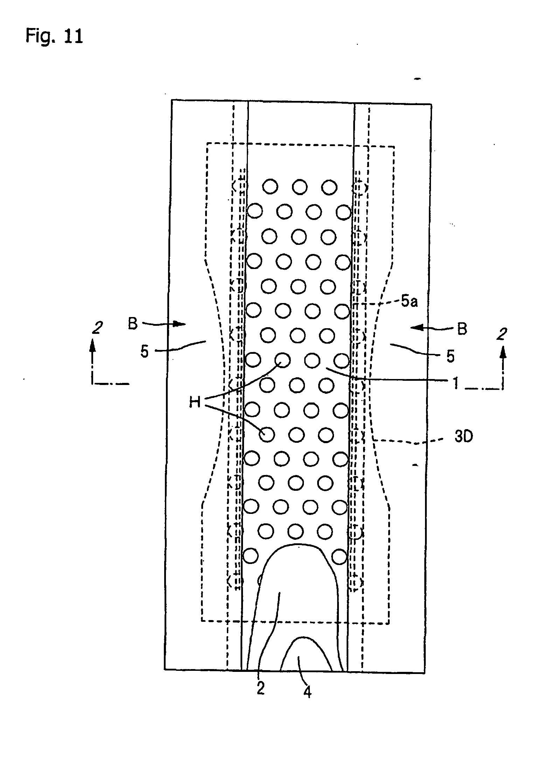

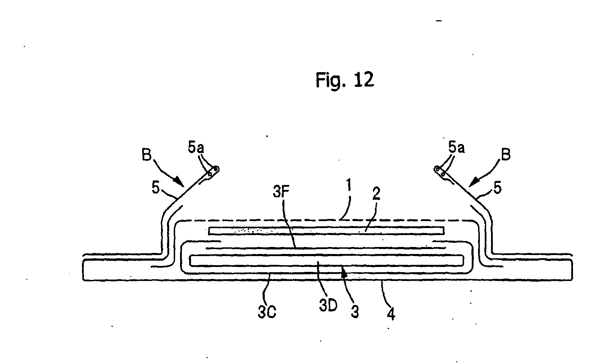

The absorbent pad shown in Fig. 11 and Fig. 12 includes in sequence from the top side toward the backside, a surface sheet 1 facing the skin, which sheet allows excremental liquid such as urine or the liquid portion of soft feces to permeate through; a permeation sheet 2 which allows excremental liquid to permeate through; and an absorbent element 3 which is disposed on the backside of the permeation sheet 2 and retains the excremental liquid. Furthermore, the absorbent pad also has barrier cuffs B, B standing toward the top surface.

The surface sheet 1 has a large number of permeation holes H, H... for excremental liquid formed at least in the area facing the anus, or over the entire area according to the embodiment. A surface sheet 1 which can be favorably used is one having an effective opening area of a single permeation hole of 3 to 75 mm$^2$, and having a ratio of opening area of 10 to 80%, with regard to a group of permeation holes for excremental liquid formed at least in the area facing the anus. If the opening area and the ratio of opening area are small, the permeability of the solid portions of soft feces is deteriorated, while if the opening area is excessively large, the solid portions of soft feces remaining behind on the surface of the permeation sheet 2 is contacted with the skin, thereby causing rashes on the skin or requiring troublesome works of skin wiping. If the ratio of opening area is excessively large, the strength of the system is decreased, which is undesirable.

The permeation sheet 2 which directly faces the surface sheet 1, functions as a second sheet in the depicted example. Details thereof will be described later.

The absorbent element 3 which directly faces the permeation sheet 2, has an absorber 3D mainly composed of, for example, cotton-like pulp for retaining excremental liquid; and a top layer sheet 3F which is disposed between this absorber 3D and the permeation sheet 2, and is formed from an air-through non-woven fabric. This top layer sheet 3F is composed of a wrapping material 3C made of tissue paper or crepe paper, and an element for wrapping the absorber 3D of the absorbent element 3.

The permeation sheet 2 is preferably formed from an air-through non-woven fabric, and details thereof will be described later.

The backside sheet 4 which constitutes the back surface side of the absorbent article is a liquid-impermeable sheet in the embodiment, and is formed from, for example, a plastic sheet. The backside sheet 4 may be permeable to air. The backside sheet 4 is extended out from the side edges of the absorbent element 3 to the lateral direction, and reaches the top surface side while being folded, thus to form flaps which cover the lateral sides of the surface sheet 1.

[0088] - At the lateral sides of the backside sheet 4 is provided a barrier sheet 5. The outer side of the barrier sheet is fixed to the extended portion of the backside sheet 4 by a hot melt adhesive or the like, and the tip side of the inner free part is folded toward the inside, with an elastic stretch member 5a such as rubber thread is fixed onto the folded part, while being stretched. Thus, upon wearing the article, the free part constitutes the standing barrier cuffs B, B. According to the present invention, it is not limited as to whether the barrier cuffs are present or not, but it is desirable to provide barrier cuffs in order to prevent side leakage of soft feces.

Subsequently, various other embodiments will be described.

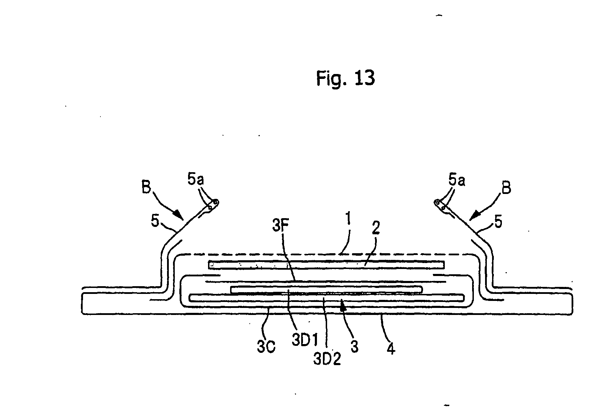

Fig. 13 shows an article in which, as the absorber 3D, a single layer absorber is replaced by an upper layer absorber 3D1 with a narrow width and a lower layer absorber 3D2, which is an other layer absorber with a broad width.

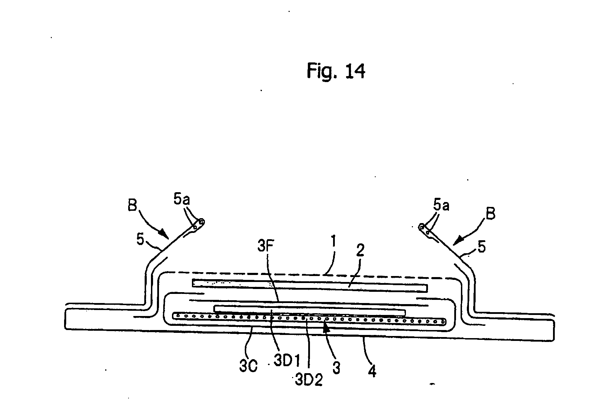

At this point, in order to retain large amounts of liquid portions in an appropriate area beneath the top layer sheet 3F of the absorbent element 3, super absorbent polymer particles (SAP) are provided. For this configuration, the particles may be dispersed within the absorber 3D, in addition to dispersing and spraying on the absorber 3D. However, in the present embodiment, since the top layer sheet 3F constituting the element for wrapping the absorber 3D is formed not from crepe paper or the like, but from an air-through non-woven fabric, there is a risk that the polymer particles may escape to the outside through the top layer sheet 3, if such configurations are employed (the configuration in which particles are dispersed and sprayed on the absorber 3D, and the configuration in which the particles are dispersed within the absorber 3D). There, as shown in Fig. 14, it is preferable to employ a bilayer structure having an upper layer absorber 3D1 and a lower layer absorber 3D2, and to incorporate super absorbent polymer particles into the lower layer absorber 3D2 only, without incorporating super absorbent polymer particles into the upper layer absorber 3D1. The objects indicated by symbol O in the diagram are super absorbent polymer particles.

From the viewpoint of preventing the escape of the super absorbent polymer particles to the outside, the upper layer absorber 3D1 preferably has a thickness of 1 to 50 mm, and a basis weight of 50 to 300 g/m$^2$. Also, since the present embodiment employs a configuration in which the wrapping material 3C of crepe paper or the like is turned in up to the upper lateral sides of the absorbent element 3, the upper layer absorber 3D1 is set to have a narrower width than the lower layer absorber 3D2 (this configuration is preferable from the viewpoint of production costs). However, if a configuration in which the top layer sheet 3F constitutes the upper lateral sides of the absorbent element 3 is to be taken, it is desirable that the upper layer absorber 3D1 is set to have a broader width than the lower layer absorber 3D2.

Although the absorber 3D is divided into two layers in the depicted example, the absorber may also be divided into three layers of an upper layer absorber, a mid-layer absorber and a lower layer absorber, or into a plurality of 4 or more, such as an upper layer absorber, a plurality of mid-layer absorbers, and a lower layer absorber. Also, it is also possible to employ a configuration in which the absorber 3D is not divided into plural layers as described above, but super absorbent polymer particles are incorporated only into the middle part or the lower part in the thickness direction of one absorber.

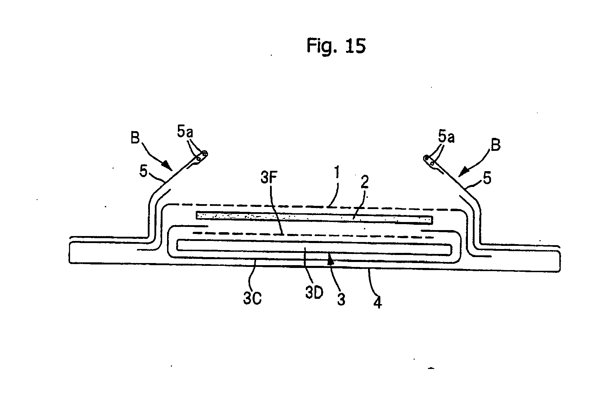

The top layer sheet 3F can be formed from a perforated sheet, as shown in Fig. 15. In this case, it is preferable, from the viewpoint of the permeability of excremental liquid, that the effective opening area of a single permeation hole is 0.5 to 40 mm$^2$, and the ratio of opening area is 1 to 80%. However, in the case of forming the top layer sheet 3F from a perforated sheet, it is preferable to provide the upper layer absorber 3D1, which does not contain the super absorbent polymer particles, to be thick, in order to prevent the super absorbent polymer particles from escaping to the outside. Also, in the depicted example, the two edge parts of the top layer sheet 3F overlap on the lower side of the side edges of the wrapping material 3C such as crepe paper, but the two edge parts can also be made to overlap on the upper side. However, in the present embodiment, since the top layer sheet 3F is made of an air-through non-woven fabric, and the excremental liquid can now easily spread in the planar direction, it is preferable to employ the former configuration (the configuration of overlapping on the lower side), in which the excremental liquid is spread within the absorbent element 3, but not on the surface of the absorbent element 3.

Furthermore, it is preferable that the soft feces permeation speed S1 of the surface sheet 1, the soft feces permeation speed S2 of the permeation sheet 2, and the soft feces permeation speed S4 for the top layer sheet 3F are correlated in the relationship of S1 > S2 > S4. Absorbent articles of this kind are required to have a good capability of carrying away soft feces rapidly from the skin as well as accommodating and retaining the soft feces at a location distant from the skin. It has been found that in order to achieve this, it is preferable that each of the constituent elements not only has a high soft feces permeation speed, but also has a speed gradient as defined in the present invention with regard to the soft feces permeation speed for each of the constituent elements, and the speed gradient is dominant to the absolute value of the speed. With the soft feces permeation speed gradient as described above, the performance of absorbing and retaining urine or the liquid portion of soft feces is excellently improved.

[Constituent materials and the like]

(Surface sheet)

The material of the surface sheet 1 is preferably a non-woven fabric so as to make the sensation of the skin good, but a plastic sheet may also be used. For the shape of opening of the hole H, appropriate shapes can be employed, such as the circular shape as depicted, an elliptical shape having the major axis along the longitudinal direction, a round shape, a triangular shape, a rectangular shape, a diamond shape, or a hexagonal shape, and these holes may be arranged in large numbers, regularly or irregularly, at a portion or over the entire surface of the surface sheet. As a suitable non-woven fabric for the surface sheet 1, an air-through non-woven fabric, a resin-bonded non-woven fabric, an air-laid non-woven fabric, a spunlace non-woven fabric, a heat-rolled non-woven fabric, a spun bond non-woven fabric, or the like may be mentioned. In the case of using a non-woven fabric for the surface sheet 1, it is preferable that the fineness of the fibers constituting the non-woven fabric is 2 to 15 dtex, particularly 3 to 10 dtex, and more particularly 4 to 10 dtex, from the viewpoint of increasing the permeability by maintaining the space for absorbing soft feces. Also, the basis weight is preferably 5 to 45 g/m$^2$. Moreover, it is preferable to use water-repellent or hydrophobic fibers for the surface sheet 1, in order to suppress diffusion on the surface sheet 1.

(Permeation sheet)

The permeation sheet 2 functions as a layer for rapidly transferring the excremental liquid which has permeated through the surface sheet 2, to the absorbent element 3. As the permeation sheet 2, a sheet mainly composed of water-repellent or hydrophobic fibers is suitable, and a non-woven fabric sheet such as an air-through non-woven fabric can also be used. However, an assembly of filaments in particular, for example, a product obtained by opening tows of bundled filaments into a sheet form, particularly a product in which filaments are oriented in the planar direction of the sheet, or a product in which filaments are spirally crimped, is preferred.

In a product obtained by opening hydrophilic tows of acetate or the like into a sheet form, the permeability of soft feces is poor. While water-repellent or hydrophobic fibers have excellent permeability for the liquid portion of soft feces, for example, an air-through non-woven fabric of PET does not have sufficient permeability for the liquid portion of soft feces. In this regard, when a product obtained by opening tows of water-repellent or hydrophobic fibers into a sheet form, is used as the permeation sheet, a permeation sheet having remarkably excellent permeability for the liquid portion of soft feces may be obtained.

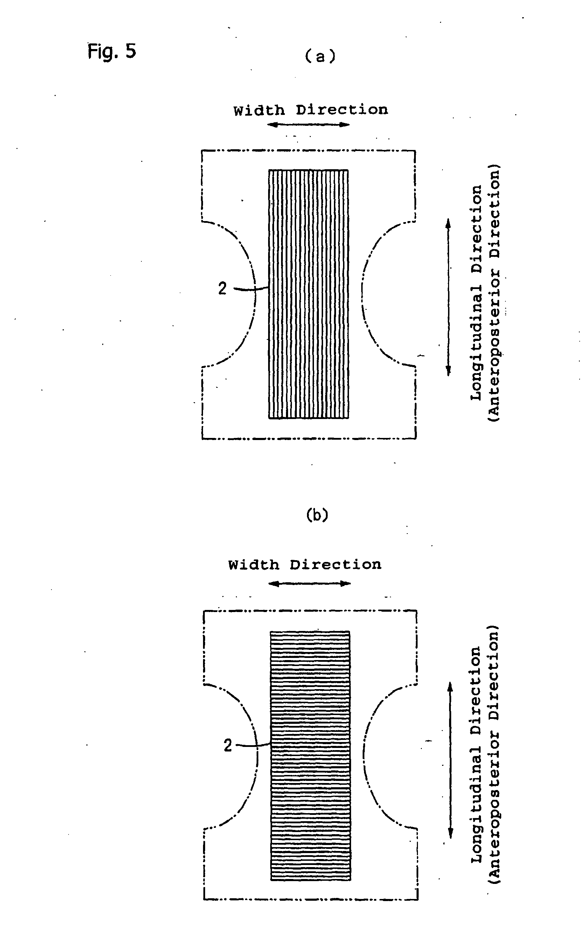

In the case where the filaments of the permeation sheet 2 are oriented in the planar direction of the sheet, the filaments can be oriented in the anteroposterior direction of the product as shown in Fig. 5(a), as well as can be oriented in the width direction (a direction perpendicular to the anteroposterior direction) of the product as shown in Fig. 5(b). The effect of allowing the liquid portion of soft feces to permeate while diffusing in the vicinities, is more significant in the case of orienting the filaments in the longitudinal direction of the product.

As the water-repellent or hydrophobic filaments that can be suitably used, fibers of PET, PP, PE and the like, as well as filaments made from those fibers into a core-in-sheath structure, may be mentioned.

The fineness of the filaments can be set to, for example, about 1 to 16 deniers, preferably 1 to 10 deniers, and more preferably 2 to 8 deniers. The filaments may be non-crimped fibers, but are preferably crimped fibers. The crimp rate of crimped fibers can be set to, for example, about 5 to 75 crimps, preferably 10 to 50 crimps, and more preferably 15 to 50 crimps, per inch. Also, crimped fibers prepared by uniform crimping are frequently used. When crimped fibers are used, a permeation sheet having a large volume and a light weight can be produced, and also highly integrated tows due to intertwining of fibers can be easily produced. The shape of cross-section of tow composing fibers (filaments) is not particularly limited, and for example, may be any of circular, elliptical, non-circular (e.g. Y-shape, X-shape, I-shape, R-shape) and hollow shapes. The filaments can be used in the form of tows (fiber bundles or filament bundles) formed by bundling, for example, about 3,000 to 1,000,000 short fibers, and preferably 5,000 to 1,000,000 short fibers. It is preferable that a tow is constructed by bundling about 3,000 to 1,000,000 filaments.

Since a tow is weak in the intertwining of fibers, a binder having an effect of adhering or fusing the contacting parts of fibers can be used for the purpose of mainly maintaining the shape. As for the binder, various resin adhesives, and particularly thermoplastic resins, can be used.

The filament assemblies can be produced by known methods. A representative example thereof involves, as previously described, forming a tow by bundling filaments, and-opening the tow. The width of an open tow can be any width, and for example, can be about 100 to 2000 mm, and preferably 150 to 1500 mm, in width. By adjusting the degree of tow opening, the density of the filament assembly can be adjusted. As for the filament assembly, one having a fiber density of 0.0075 g/cm$^3$ or less, and particularly 0.0060 to 0.0070 g/cm$^3$, with a thickness of 10 mm, is very suitable. If the fiber density is excessively increased, it becomes less benefited to use fiber assemblies formed from tows, and thus the permeability for the liquid portion of soft feces is decreased. Also, the basis weight of the permeation sheet of the present invention is preferably 10 to 100 g/m$^2$. The basis weight of the permeation sheet 2 can be adjusted by the selection of the tow to form the original fabric, of by the production conditions.

As the method of opening tows, for example, a method of loading a tow to a plurality of tow opening rolls, and gradually enlarging the width of the tow along with the proceeding of the tow; a method of opening a tow by repeating tensing (stretching) and relaxing (contraction) of the tow; a method of widening and opening a tow using compressed air; or the like may be used.

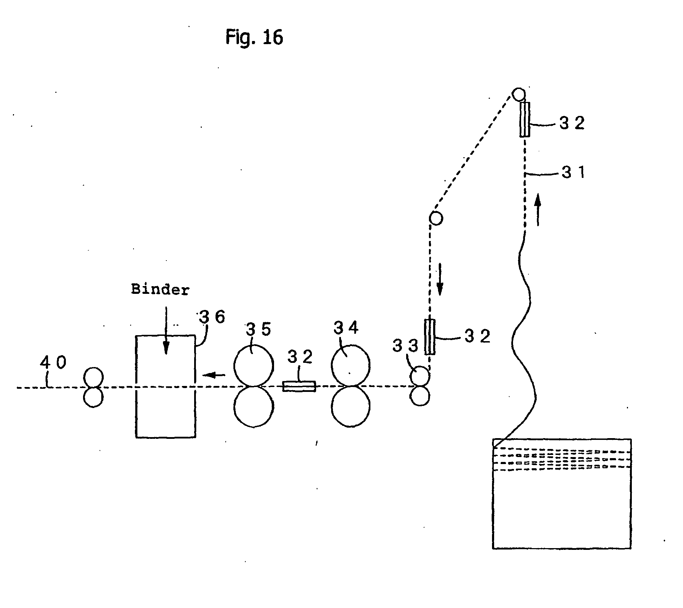

Fig. 16 is a schematic diagram showing an exemplary tow opening facility. In this example, a tow 31 which will serve as the original fabric, is sequentially drawn out, and during the conveying process, is passed through a tow opening unit which combines a widening means 32 using compressed air, and a plurality of tow opening nip rolls 33, 34 and 35 having a peripheral speed as fast as the rolls in the downstream, so as to be widened and opened. Then, a binder is added to the tow through a binder adding box 36, and the tow is formed into a fiber bundle 40 of filaments with desired width and density. In the case of applying to the absorbent article of the present embodiment, it is desirable to use the filaments after cutting the filaments to have desired shape and dimension.

The permeation sheet 2 is preferably of a single sheet structure, but if necessary, may have a laminate structure, so that the sheet is considered substantially as a single sheet. As the lower layer sheet in this case, an air-through non-woven fabric, a resin-bonded non-woven fabric, an air-laid non-woven fabric, a spunlace non-woven fabric, a heat-rolled non-woven fabric, a spun bond non-woven fabric or the like may be used, but an air-through non-woven fabric is most appropriate. Among the air-through non-woven fabrics, those using water-repellent or hydrophobic fibers are particularly preferred. As the water-repellent or hydrophobic fibers, fibers of PET, PP, PE and the like, and fibers made of these materials and having a core-in-sheath structure may be mentioned. As the lower layer sheet, a perforated sheet can be used. In this case, it is preferable that the effective opening area of a single permeation hole is 0.5 to 40 mm$^2$, and the ratio of opening area is 1 to 80%, from the viewpoint of the permeability of liquid portion.

The basis weight of a single permeation sheet 2, or a permeation sheet 2 which is substantially considered as a single sheet, is preferably 10 to 60 g/m$^2$.

(Absorbent element) -

[Configuration 1: Embodiments of the invention according to claims 1 to 4]

As the absorbent element 3 which directly faces the permeation sheet 2, various configurations as described above can be employed, as long as the cotton-like pulp layer directly faces the permeation sheet 2.

As the cotton-like pulp layer, known cotton-like pulp can be used, and the cotton-like pulp layer can be produced by integrating pulp fibers by a known method, and interlacing the pulp fibers by means of air, a water stream or the like. As the pulp fibers, those comprising cellulose fibers such as chemical pulp obtained from wood or dissolved pulp, or artificial cellulose fibers such as rayon or acetate, can be used. Hardwood pulp having a longer fiber length, rather than softwood pulp, is suitably used from the aspects of function and price.

Furthermore, instead of incorporating super absorbent polymer in the cotton-like pulp layer 3A1 and the cotton-like pulp layer 3A2, it is also possible to provide a layer of super absorbent polymer on at least one side of the front surface side and the back surface side of the cotton-like pulp layer 3A1 and the cotton-like pulp layer 3A2. In this case, the super absorbent polymer can be adhered to the wrapping material 3B which wraps the cotton-like pulp layer 3A1 and the cotton-like pulp layer 3A2. As the adhesive for this case, the thermoplastic resins described above can be used.

[Configuration 2: Embodiments of the invention according to claims 5 to 8]

As the upper side wrapping material 3E, an air-through non-woven fabric, a resin-bonded non-woven fabric, an air-laid non-woven fabric, a spunlace non-woven fabric, a heat-rolled non-woven fabric, a spun bond non-woven fabric and the like can be used, but an air-through non-woven fabric is most suitable. Among the air-through non-woven fabrics, it is particularly preferable to use water-repellent or hydrophobic fibers. As the water-repellent or hydrophobic fibers, fibers of PET, PP, PE and the like, as well as fibers made of these and having a core-in-sheath structure may be mentioned.

The basis weight of the upper side wrapping material is preferably 10 t0 60 g/m$^2$. The upper side wrapping material 3E can be made into a single layer, as well as can be laminated to a structure of two layers or more, for example a trilayer structure. If laminated, the basis weight in the laminated state is preferably 10 to 60 g/m$^2$.

The upper side wrapping material 3A can be formed from a perforated sheet. In this case, it is preferable that the effective opening area of a single permeation hole is 0.5 to 40 mm$^2$, and the ratio of opening area is 1 to 80%, from the viewpoint of the permeability of liquid portion. In the case where the wrapping material is a perforated sheet, perforation may be performed on various non-woven fabrics, or a plastic sheet may also be perforated.

As the absorbers 3D1 and 3D2, known cotton-like pulp can be used, and the absorbers can be produced by integrating pulp fibers by a known method, and interlacing the pulp fibers by means of air, a water stream or the like. As for the pulp fibers, those comprising cellulose fibers such as chemical pulp obtained from wood or dissolved pulp, or artificial cellulose fibers such as rayon or acetate, can be used. Hardwood pulp having a longer fiber length, rather than softwood pulp, is suitably used from the aspects of function and price.

In the part wrapped by the upper side wrapping material 3E and the lower side wrapping material 3C of the absorbent element 3, super absorbent polymer particles (SAP) can be encapsulated for the purpose of retaining large quantities of liquid portions.

Furthermore, instead of incorporating super absorbent polymer in the absorbers 3D1 and 3D2, it is also possible to provide a layer of super absorbent polymer on at least one side of the front surface side and the back surface side of the absorbers 3D1 and 3D2. In this case, the super absorbent polymer can be adhered to the wrapping materials 3E and 3C, which wrap the absorbers 3D1 and 3D2. As the adhesive for this case, the thermoplastic resins described above can be used.

[Configuration 3: Embodiments of the invention according to claims 9 to 14]

For the element for wrapping the absorber 3D, at least a portion of the area located between the absorber 3D_and the permeation sheet 2 (top layer sheet 3A), preferably the area facing the anus, and more preferably the entire region, is formed not from crepe paper or the like, but from a non-woven fabric having a density of 0.01 to 0.2 $g/cm^3$, or an air-through non-woven fabric. In the absorbent articles of prior art, since the element for wrapping the absorber 3D have all been composed of crepe paper or the like, a viscous excremental liquid would form a membrane (barrier) on the surface of the crepe paper or the like, causing inhibition of the permeation of urine or soft feces. However, if the area located between the absorber 3D and the permeation sheet 2 is formed from a non-woven fabric having a predetermined density or an air-through non-woven fabric, the excremental liquid such as the liquid portion of soft feces can be transferred to the absorber 3D rapidly and surely. In particular, if the density of the non-woven fabric is 0.01 $g/cm^3$ or greater, any risk that the non-woven fabric would be torn off, and surperabsorbent polymer particles would spill out, is completely eliminated. Also, if the density of the non-woven fabric is 0.2 $g/cm^3$ or less, any risk that the non-woven fabric would cause inhibition of the permeation of urine or soft feces, is completely eliminated. Of course, even if crepe paper is changed to these non-woven fabrics, the performance of maintaining the shape of the absorber 3D is maintained the same as in the case of crepe paper.

Here, the density ($g/cm^3$) of the non-woven fabric is taken from the value determined from the basis weight ($g/cm^2$) /thickness (cm). The thickness is the value obtained by measuring under a load of 0.7 kPa, based on JIS P8118.

Among the non-woven fabrics, it is particularly preferable to use not hydrophilic fibers, but water-repellent or hydrophobic fibers, for the same reasons as in the case of the permeation sheet 2 described above. As the water-repellent or hydrophobic fibers, fibers of PET, PP, PE and the like, as well as fibers made of these and having a core-in-sheath_structure may be mentioned.

The top layer sheet 3A is preferably one having a fineness of 2 to 7 dtex and a basis weight of 10 to 45 $g/m^2$, and more preferably one having a fineness of 3.3 to 5.6 dtex and a basis weight of 20 to 40 $g/m^2$. If this condition is satisfied, the permeability of viscous excremental liquid is secured, and also the risk of super absorbent polymer spilling out is reduced. Thus, the upper layer absorber 3D1 described above can be omitted. At this point, from the viewpoint of the permeability of viscous excremental liquid, a sheet having a large fineness and a small basis weight is preferred (that is, a sheet in a sieve form, rather than of the reticulate shape of wire screen, is preferred). However, if this is brought further, the super absorbent polymer becomes prone to spill out to the outside. Thus, this balance is established by means of the fineness and the basis weight. In addition, if the basis weight is less than $10^9/m^2$, the strength of the sheet is weakened.

The top layer sheet 3A can be of a single layer, or can be laminated into bilayers, trilayers, quadrilayers or more multiple layers.

Here, as the absorber 3D, for example, cotton-like pulp or the same material as the permeation sheet 2 can be used. The cotton-like pulp can be produced by integrating pulp fibers by a known method, and interlacing the pulp fibers by means of air, a water stream or the like. As the pulp fibers, those comprising cellulose fibers such as chemical pulp obtained from wood or dissolved pulp, or artificial cellulose fibers such as rayon or acetate, can be used. Hardwood pulp having a longer fiber length, rather than softwood pulp, is suitably used from the aspects of function and price.

(Super absorbent polymer)

As the super absorbent polymer particles (SAP) that can be used, for example, in the above-described form for the absorbent element 3, carboxymethylcellulose, polyacrylic acid and salts thereof, acrylic acid salt polymer cross-linking products, starch-acrylic acid graft copolymers, hydrolysates of starch-acrylonitrile graft copolymers, polyoxyethylene cross-linking products, carboxymethylcellulose cross-linking products, partial cross-linking products of water-swellable polymers such as polyethylene oxide or polyacrylamide, copolymers of isobutylene and maleic acid, and the like are suitably used. In order to suppress the blocking properties due to moisture absorption of the product, polymers having blocking preventing agents added can also be used. Also, as the super absorbent polymers, those in various forms such as powdered, particulate, granular, pellet-like, sol-like, suspension-like, gel-like, film-like, or non-woven fabric-like forms, are available. However, these all can be used for the present invention, and particularly powdered product is very suitably used.

As the method of incorporating a super absorbent polymer, a method of conferring by spraying a super absorbent polymer in the powdered particle form on the exterior or interior of the absorber; a method of impregnating the absorber with a monomer (to be a super absorbent polymer) and then polymerizing the monomer; a method of coating a non-cross-linked, gel-like super absorbent polymer on the absorber; and the like can be employed.

The basis weight of the super absorbent polymer can be appropriately set in accordance with the amount of absorption required in response to the amount of excremental liquid. Therefore, although it cannot be defined in short words, the basis weight can be set generally to 0.03 g/cm$^2$ or less, and particularly suitably to 0.01 to 0.025 g/cm$^2$.

(Backside sheet 4)

The backside sheet 4 constituting the backside of the absorbent article is a liquid-impermeable sheet in the embodiment, and for example, a plastic sheet can be used. The backside sheet 4 may be permeable to air.

EXAMPLE 1

(Experimental Example 1)

Pseudo-feces resembling soft feces were prepared, and the permeation speed of the pseudo-feces in a structure which was similar to the product as much as possible, was investigated. The composition of the pseudo-feces was as follows.

| | |
|---|---|
| Component 1. Starch (potato starch) | 6% |
| Component 2. Starch (buckwheat starch) | 3% |
| Component 3. Powdered grind stone (aluminum silicate) | 3% |
| Component 4. Artificial urine | 88% |
| Surfactant | small amount |

At the time of preparation, a liquid having the above-described composition was prepared by (1) introducing component 1 into the artificial urine of component 4, and heating the mixture to 100°C while stirring, to dissolve the starch; (2) cooling the temperature of the product of (1) to room temperature, and then introducing component 2; (3) introducing component 3 in the same manner as in (2); and (4) if uniform diffusion is confirmed, adding the surfactant and adjusting the liquid to 28 dyne.

For the absorbent pad having the configuration of Fig. 1 and Fig. 2, a spun bond non-woven fabric made of PP, which had permeation holes for excremental liquid having a size of a major axis of 4 mm and a minor axis of 3 mm, disposed in a zigzag pattern at a separation distance of 1.5 mm in the 45° width direction and the longitudinal direction, and had a basis weight of 35 g/m$^2$, was used as the surface sheet.

For each of these samples to be compared, a cylinder having a diameter of 3 inches was installed on the surface sheet, and 50 cc of the above-described pseudo-feces (colored product) was injected continuously at a speed of 15 cc/sec. After the initiation of this injection, the time (seconds) taken until the presence of the pseudo-feces could not be recognized by naked eyes, was taken as the absorption speed. Also, after the absorption, it was qualitatively determined as to whether the strike-through properties were good or poor. The results are presented in Table 1.

[Table 1]

| | Comparative Example 1 | Comparative Example 2 | Comparative Example 3 | Experimental Example |
|---|---|---|---|---|
| Permeable sheet | | | | |
| Production method | Sheet prepared by tow opening (filament assembly) | Sheet prepared by tow opening (filament assembly) | Air-through non-woven fabric | Sheet prepared by tow opening (filament assembly) |
| Type of fiber | Acetate | Acetate | PET/PE | PET/PE |
| Hydrophilic/ Water-repellent | Hydrophilic | Hydrophilic | Water-repellent | Water-repellent |
| Fiber size | 4d | 4d | 3d | 3d |
| Base weight | 50g/m$^2$ | 100g/m$^2$ | 50g/m$^2$ | 50g/m$^2$ |
| Absorption speed (sec) | 600< | 600< | 600< | 20 |

(continued)

| | Comparative Example 1 | Comparative Example 2 | Comparative Example 3 | Experimental Example |
|---|---|---|---|---|
| Strike-through evaluation | × | × | × | ○ |

From the results of Table 1, it was proved that the absorption speed was slow with filament assemblies prepared by opening hydrophilic tows, and water-repellent air-through non-woven fabrics. On the contrary, it was found that the use of hydrophilic filament assemblies prepared by tow opening for the permeation sheet resulted in a notably fast absorption speed.

In addition, it was found that surface sheets having an opening area of 3 mm$^2$ or less (including non-perforated sheets) all had poor permeability for the pseudo-feces, even though the ratio of opening area or the material was changed in any way.

Industrial Applicability

The present invention can be applied as an absorbent article such as a disposable diaper or an absorbent pad, and particularly as an absorbent article having excellent performance of absorbing and retaining viscous excremental liquid such as soft feces.

Brief Description of the Drawings

Fig. 1 is a plane view of an exemplary absorbent article according to the present invention, in an expanded state.
Fig. 2 is an arrow cross-sectional view of Fig. 1 along the line 2-2.
Fig. 3 is a cross-sectional view of another configuration.
Fig. 4 is a cross-sectional view of another configuration.
Fig. 5 is a plane view of an absorbent article in an expanded state, for explaining the orientation form_of the filaments constituting the permeation sheet.
Fig. 6 is an explanatory diagram illustrating the bonding form of the surface sheet to the permeation sheet.
Fig. 7 is an explanatory diagram of an exemplary relationship between the permeation holes of the surface sheet and the fusion section.
Fig. 8 is a plane view of an exemplary absorbent article according to the present invention, in an expanded state.

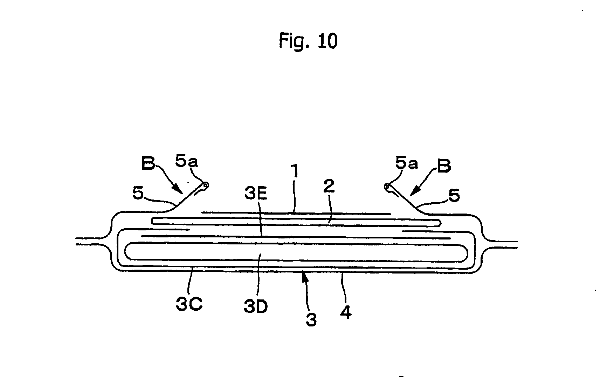

Fig. 9 is an arrow cross-sectional view of Fig. 8 along the line 2-2. Fig. 10 is a cross-sectional view of another configuration.
Fig. 11 is a cross-sectional view of another configuration.
Fig. 12 is an arrow cross-sectional view of Fig. 11 along the line 2-2.
Fig. 13 is a cross-sectional view of another configuration.
Fig. 14 is a cross-sectional view of another configuration.
Fig. 15 is a cross-sectional view of still another configuration.
Fig. 16 is a schematic explanatory diagram of the method of tow opening in the case of obtaining a permeable sheet.

Reference Numerals

1: Surface sheet
2: Permeation sheet
2S: Exposed part
3: Absorbent element
3A: Cotton-like pulp layer
3A1: Upper cotton-like pulp layer
3A2: Lower cotton-like pulp layer
3B: Crepe paper
3C: Lower side wrapping material

3D: Absorber
3D1: Upper side absorber
3D2: Lower side absorber
3E: Upper side wrapping material
3F: Top layer sheet
4: Backside sheet
4a: Elastic stretch member
5: Barrier sheet
5a, 5b: Elastic stretch members
B: Barrier cuffs
BE: Back edge part
C: Middle part
d: Quasi-fusion section
F: Fixed region
FE: Front edge part
H: Permeation hole
m: Fusion section
S1-4: Soft feces permeation speed

**Claims**

1. An absorbent article including in sequence, a perforated surface sheet (1) facing the skin, when in use, and allows permeation of excremental liquid therethrough; a permeation sheet (2) which allows permeation of excremental liquid therethrough; and an absorbent element (3) disposed on a backside of the permeation sheet (2) which includes cotton-like pulp for retaining the excremental liquid, wherein

   - a soft feces permeation speed S1 of the surface sheet (1) is higher than a soft feces permeation speed S2 of the permeation sheet (2);
   - the perforated surface sheet (1) has a group of permeation holes (H) for excremental liquid formed at an excretion region thereof where at least an area that faces, when in use, the body part excreting the excremental liquid and its vicinity are included, and an effective opening area of a single permeation hole (H) is 3 to 75 mm$^2$, and a ratio of opening area is 10 to 80%;
   - the absorbent element (3) has a cotton-like pulp layer (3A) exposed on a use side thereof, and the surface sheet (1), the permeation sheet (2) and the cotton-like pulp layer (3A) of the absorbent element (3) are directly adjacent in sequence,
   - the article has a main body section which includes the permeation sheet (2), the absorbent element (3) and a fusion section (m) formed by fusing the surface sheet (1) onto a top surface of the main body section, wherein the fusion section (m) is provided at at least one site in the width direction at various locations in the front and rear directions of the article, and
   - the fusion section (m) is formed to have a longer length in the MD direction and in the CD direction, compared to the permeation holes (H) of the surface sheet (1).

2. The absorbent article according to claim 1, wherein the soft feces permeation speed S2 of the permeation sheet is greater than a soft feces permeation speed S3 of the cotton-like pulp layer (3A) of the absorbent element (3).

3. The absorbent article according to claim 1 or 2, wherein the permeation sheet (2) is made mainly of water-repellent or hydrophobic fibers.

4. The absorbent article according to any one of claims 1 to 3, wherein the permeation sheet (2) comprises a filament assembly, and filaments of the filament assembly are oriented in the planar direction of the sheet.

5. The absorbent article according to any one of claims 1 to 4, wherein an area ratio of the fusion section (m) is lower in the middle part (C) between front and back edges (FE, BE) than that at the front and back edges (FE, BE) of the article.

6. The absorbent article according to claim 5, wherein the main body section is exposed at both lateral sides of the surface sheet (1), and a quasi-fusion section (d) is formed on an exposed part of the main body section by subjecting an upper surface of the main body section to fusion-processing without involving the surface sheet (1).

7. The absorbent article according to any one of claims 1 to 6, wherein the absorbent element (3) has an absorber 3D) containing super absorbent polymer particles and an element for wrapping the absorber (3D), and at least part of an area of the element for wrapping the absorber (3D) located between the absorber (3D) and the permeation sheet (2) is made of a non-woven fabric having a density of 0.01 to 0.2 g/cm$^3$.

8. The absorbent article according to any one of claims 1 to 7, wherein the absorbent element (3) has an absorber (3D) containing super absorbent polymer particles and an element for wrapping the absorber (3D), and at least part of an area of the element for wrapping the absorber (3D) located between the absorber (3D) and the permeation sheet (2) is made of an air-through non-woven fabric.

9. The absorbent article according to claim 7 or 8,
wherein an area of the element for wrapping the absorber (3D) other than the area made of a non-woven fabric is made of tissue paper or crepe paper (3B).

10. The absorbent article according to any one of claims 7 to 9, wherein the non-woven fabric has a fineness of 2 to 7 dtex and a basis weight of 10 to 45 g/m$^2$.

11. The absorbent article according to any one of claims 7 to 10, wherein the non-woven fabric comprises water-repellent or hydrophobic fibers.

12. The absorbent article according to any one of claims 7 to 11, wherein the absorber comprises an upper layer absorber (3D1) and another layer absorber (3D2), and the upper layer absorber (3D1) does not contain super absorbent polymer particles.

**Patentansprüche**

1. Saugfähiger Artikel, der der Reihe nach folgende Lagen umfasst: eine perforierte Oberflächenlage (1), die bei Gebrauch der Haut zugewandt ist, und eine Permeation von Exkrementflüssigkeit zulässt; eine Permeationslage (2), die eine Permeation von Exkrementflüssigkeit zulässt; und ein saugfähiges Element (3), das an einer Rückseite der Permeationslage (2) angeordnet ist, das einen baumwollartigen Zellstoff zum Zurückhalten der Exkrementflüssigkeit aufweist, wobei

- eine Permeationsgeschwindigkeit S1 für weichen Kot der Oberflächenlage (1) höher als eine Permeationsgeschwindigkeit S2 für weichen Kot der Permeationslage (2) ist;
- die perforierte Oberflächenlage (1) eine Gruppe von Permeationslöchern (H) für Exkrementflüssigkeit aufweist, die bei einem Ausscheidungsbereich gebildet sind, wobei wenigstens eine Fläche, die im Gebrauch dem Körperteil zugewandt ist, das die Exkrementflüssigkeit ausscheidet, und deren Umgebung enthalten sind, und wobei eine wirksame Öffnungsfläche eines einzelnen Permeationslochs (H) 3 bis 75 mm$^2$ beträgt, und wobei ein Anteil einer Öffnungsfläche 10 bis 80% beträgt;
- das saugfähige Element (3) eine baumwollartige Zellstofflage (3A) aufweist, die auf einer Verwendungsseite freiliegt, und wobei die Oberflächenlage (1), die Permeationslage (2) und die baumwollartige Zellstofflage (3A) des saugfähigen Elements (3) der Reihe nach direkt aneinander angrenzen,
- der Artikel einen Hauptkörperabschnitt aufweist, der die Permeationslage (2), das saugfähige Element (3) und einen Verbindungsabschnitt (m), der durch Verbinden der Oberflächenlage (1) auf einer oberen Fläche des Hauptkörperabschnitts gebildet wird, aufweist, wobei der Verbindungsabschnitt (m) an wenigstens einer Stelle in der Breitenrichtung an verschiedenen Positionen in der vorderen und hinteren Richtung des Artikels vorgesehen ist, und
- der Verbindungsabschnitt (m) so gebildet ist, dass er im Vergleich zu den Permeationslöchern (H) der Oberflächenlage (1) in der MD-Richtung und der CD-Richtung eine längere Länge aufweist.

2. Saugfähiger Artikel nach Anspruch 1, wobei die Permeationsgeschwindigkeit S2 für weichen Kot der Permeationslage größer als eine Permeationsgeschwindigkeit S3 für weichen Kot der baumwollartigen Zellstofflage (3A) des saugfähigen Elements (3) ist.

3. Saugfähiger Artikel nach Anspruch 1 oder 2, wobei die Permeationslage (2) hauptsächlich aus wasserabweisenden oder hydrophoben Fasern hergestellt ist.

4. Saugfähiger Artikel nach einem der Ansprüche 1 bis 3, wobei die Permeationslage (2) eine Filamentanordnung umfasst und wobei Filamente der Filamentanordnung in der planaren Richtung der Lage orientiert sind.

5. Saugfähiger Artikel nach einem der Ansprüche 1 bis 4, wobei ein Flächenanteil des Verbindungsabschnitts (m) in dem mittleren Teil (C) zwischen einer vorderen und einer hinteren Kante (FE, BE) kleiner als bei der vorderen und der hinteren Kante (FE, BE) des Artikels ist.

6. Saugfähiger Artikel nach Anspruch 5, wobei der Hauptkörperabschnitt an beiden seitlichen Seiten der oberen Lage (1) freiliegt und wobei ein verbindungsartiger Abschnitt (d) an einem freiliegenden Teil des Hauptkörperabschnitts gebildet wird, indem eine obere Fläche des Hauptkörperabschnitts einem Verbindungsverfahren ohne Einbeziehung der Oberflächenlage (1) unterzogen wird.

7. Saugfähiger Artikel nach einem der Ansprüche 1 bis 6, wobei das saugfähige Element (3) ein Absorptionsmittel (3D) aufweist, das superabsorbierende Polymerpartikel und ein Element zum Umwickeln des Absorptionsmittels (3D) umfasst, und wobei wenigstens ein Teil einer Fläche des Elements zum Umwickeln des Absorptionsmittels (3D), die sich zwischen dem Absorptionsmittel (3D) und der Permeationslage (2) befindet, aus einem Faservliesstoff mit einer Dichte zwischen 0,01 und 0,2 g/cm$^3$ hergestellt ist.

8. Saugfähiger Artikel nach einem der Ansprüche 1 bis 7, wobei das saugfähige Element (3) ein Absorptionsmittel (3D) aufweist, das superabsorbierende Polymerpartikel und ein Element zum Umwickeln des Absorptionsmittels (3D) umfasst, und wobei wenigstens ein Teil einer Fläche des Elements zum Umwickeln des Absorptionsmittels (3D), die sich zwischen dem Absorptionsmittel (3D) und der Permeationslage (2) befindet, aus einem luftdurchlässigen Faservliesstoff hergestellt ist.

9. Saugfähiger Artikel nach Anspruch 7 oder 8, wobei eine Fläche des Elements zum Umwickeln des Absorptionsmittels (3D) im Gegensatz zu der Fläche, die aus einem Faservliesstoff hergestellt ist, aus Tissue-Papier oder Krepppapier (3B) hergestellt ist.

10. Saugfähiger Artikel nach einem der Ansprüche 7 bis 9, wobei der Faservliesstoff eine Feinheit von 2 bis 7 dtex und ein Basisgewicht von 10 bis 45 g/m$^2$ aufweist.

11. Saugfähiger Artikel nach einem der Ansprüche 7 bis 10, wobei der Faservliesstoff wasserabweisende oder hydrophobe Fasern umfasst.

12. Saugfähiger Artikel nach einem der Ansprüche 7 bis 11, wobei das Absorptionsmittel ein obere Absorptionsmittellage (3D1) und eine weitere Absorptionsmittellage (3D2) umfasst und wobei die obere Absorptionsmittellage (3D1) keine superabsorbierenden Polymerpartikel enthält.

**Revendications**

1. Article absorbant comprenant, dans l'ordre : une feuille de surface perforée (1) qui fait face à la peau, en service, et permet la perméation du liquide excrémentiel à travers ; une feuille de perméation (2) qui permet la perméation du liquide excrémentiel à travers ; et un élément absorbant (3) disposé sur le côté arrière de la feuille de perméation (2) qui comprend une pâte similaire à du coton destinée à retenir le liquide excrémentiel, dans lequel :

- la vitesse de perméation des selles molles S1 de la feuille de surface (1) est supérieure à la vitesse de perméation des selles molles S2 de la feuille de perméation (2) ;
- la feuille de surface perforée (1) présente un groupe de trous de perméation (H) du liquide excrémentiel, formés au niveau d'une région d'excrétion de celui-ci, où sont inclus une zone au moins qui fait face, en service, à la partie du corps qui excrète le liquide excrémentiel et son voisinage, et la surface d'ouverture utile d'un seul trou de perméation (H) est comprise entre 3 mm$^2$ et 75 mm$^2$, et le rapport de la surface d'ouverture est compris entre 10 % et 80 % ;
- l'élément absorbant (3) présente une couche de pâte similaire à du coton (3A) exposée du côté utilisation de celui-ci, et la feuille de surface (1), la feuille de perméation (2) et la couche de pâte similaire à du coton (3A) de l'élément absorbant (3), sont directement adjacentes à la suite les unes des autres ;

- l'article présente une section corps principal qui comprend la feuille de perméation (2), l'élément absorbant (3) et une section fusion (m) formée en faisant fusionner la feuille de surface (1) sur la surface supérieure de la section corps principal, dans lequel la section fusion (m) est disposée au niveau d'un emplacement au moins dans la direction de la largeur à divers emplacements dans les directions avant et arrière de l'article ; et
- la section fusion (m) est formée de façon à présenter une plus longue longueur dans la direction MD et dans la direction CD, en comparaison des trous de perméation (11) de la feuille de surface (1).

**2.** Article absorbant selon la revendication 1, dans lequel la vitesse de perméation des selles molles S2 de la feuille de perméation, est supérieure à la vitesse de perméation des selles molles S3 de la couche de pâte similaire à du coton (3A) de l'élément absorbant (3).

**3.** Article absorbant selon la revendication 1 ou la revendication 2, dans lequel la feuille de perméation (2) est constituée principalement de fibres hydrofuges ou hydrophobes.

**4.** Article absorbant selon l'une quelconque des revendications 1 à 3, dans lequel la feuille de perméation (2) comprend un ensemble de filaments, et les filaments de l'ensemble de filaments sont orientés dans la direction plane de la feuille.

**5.** Article absorbant selon l'une quelconque des revendications 1 à 4, dans lequel le rapport de surface de la section fusion (m) est inférieur dans la partie médiane (C) entre les bords avant et arrière (FE, BE), à celui au niveau des bords avant et arrière (FE, BE) de l'article.

**6.** Article absorbant selon la revendication 5, dans lequel la section corps principal est exposée au niveau des deux côtés latéraux de la feuille de surface (1), et une section quasi-fusion (d) est formée sur une partie exposée de la section corps principal en soumettant une surface supérieure de la section corps principal à un traitement de fusion sans impliquer la feuille de surface (1).

**7.** Article absorbant selon l'une quelconque des revendications 1 à 6, dans lequel l'élément absorbant (3) présente un agent absorbant (3D) qui contient des particules polymères superabsorbantes et un élément destiné à envelopper l'agent absorbant (3D), et une partie au moins d'une surface de l'élément destiné à envelopper l'agent absorbant (3D) située entre l'agent absorbant (3D) et la feuille de perméation (2), est réalisée dans un tissu non tissé qui présente une densité comprise entre 0,01 g / cm$^3$ et 0,2 g / cm$^3$.

**8.** Article absorbant selon l'une quelconque des revendications 1 à 7, dans lequel l'élément absorbant (3) présente un agent absorbant (3D) qui contient des particules polymères superabsorbantes et un élément destiné à envelopper l'agent absorbant (3D), et une partie au moins d'une surface de l'élément destiné à envelopper l'agent absorbant (3D) située entre l'agent absorbant (3D) et la feuille de perméation (2), est réalisée dans un tissu non tissé perméable à l'air.

**9.** Article absorbant selon la revendication 7 ou la revendication 8, dans lequel une surface de l'élément destiné à envelopper l'agent absorbant (3D) autre que la surface réalisée dans un tissu non tissé, est réalisée en papier de soie ou en papier de crêpe (3B).

**10.** Article absorbant selon l'une quelconque des revendications 7 à 9, dans lequel le tissu non tissé présente une finesse comprise entre 2 dtex et 7 dtex et une masse surfacique comprise entre 10 g / m$^2$ et 45 g / m$^2$.

**11.** Article absorbant selon l'une quelconque des revendications 7 à 10, dans lequel le tissu non tissé comprend des fibres hydrofuges ou hydrophobes.

**12.** Article absorbant selon l'une quelconque des revendications 7 à 11, dans lequel l'agent absorbant comprend un agent absorbant de couche supérieure (3D1) et un agent absorbant d'une autre couche (3D2), et l'agent absorbant de couche supérieure (3D1) ne contient pas de particules polymères superabsorbantes.

Fig. 1

Fig. 2

Fig. 3

Fig. 4

Fig. 5

(a)

Width Direction

Longitudinal Direction
(Anteroposterior Direction)

2

(b)

Width Direction

Longitudinal Direction
(Anteroposterior Direction)

2

Fig. 6

Fig. 7

Fig. 8

Fig. 9

Fig. 10

Fig. 11

Fig. 12

Fig. 13

# Fig. 14

Fig. 15

EP 1 908 441 B1

## Fig. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2065861 A
- JP 2001276125 A
- JP 2001269362 A
- JP 5643240 B